# EUROPEAN PATENT SPECIFICATION

(11) **EP 4 491 166 B1**
(45) Date of publication and mention of the grant of the patent: **01.07.2026**
(21) Application number: 23819781.8
(22) Date of filing: 02.06.2023
(51) Int. Cl.: A61F 13/49

(54) **STRETCHABLE SHEET AND UNDERPANTS-TYPE ABSORBENT ARTICLE**
DEHNBARE FOLIE UND SAUGFÄHIGER ARTIKEL VOM UNTERHOSEN-TYP
FEUILLE ÉTIRABLE ET ARTICLE ABSORBANT DE TYPE CULOTTE

(30) Priority: 10.06.2022 JP 2022094704
(43) Date of publication of application: 15.01.2025
(73) Proprietor: Unicharm Corporation, Ehime 799-0111 (JP)
(72) Inventor: AKINO, Chieri, Kanonji-shi, Kagawa 769-1602 (JP); NAGAI, Takahito, Kanonji-shi, Kagawa 769-1602 (JP); MUKAI, Hirotomo, Kanonji-shi, Kagawa 769-1602 (JP)
(74) Representative: Dolleymores
(86) International application number: PCT/JP2023/020676
(87) International publication number: WO 2023/238801

(56) References cited:
- WO-A1-2018/154684
- JP-A- 2019 063 411
- JP-A- 2020 536 648
- JP-A- 2021 503 351
- US-B2- 10 980 678
- US-B2- 10 987 255

## Description

### [Technical Field]

The present invention relates a stretchy sheet and an underpants-shaped absorbent article.

### [Background Art]

In stretchy sheet used in underpants-shaped absorbent articles and the like, it is common to join elastic members to a sheet material in a stretched state, and then contract the elastic members to form gathers in the sheet material. Patent Document 1 is directed to formation of creases (gathers) in a stretchy sheet, the creases extending continuously in the direction orthogonal to the stretching/contracting direction of the elastic members. To achieve this, two sheet materials sandwiching the elastic members are partially joined by heat-sealed portions arranged in a staggered pattern or by heat-sealed portions constituting a row along a direction orthogonal to the stretching/contracting direction.

Patent Document 2 discloses a stretchy sheet formed of two opposing sheets joined together by rows of welded portions, which rows extend generally along a direction orthogonal to the stretching/contracting direction of the stretchy sheet but have convex portions in the stretching/contracting direction.

### [Citation List]

### [Patent Literature]

[Patent Document 1] Japanese Patent Application Publication NO.2007-14802
[Patent Document 2] United States Patent US10980678B2

### [Summary of Invention]

### [Technical Problem]

However, as in Patent Document 1, if multiple heat-sealed portions are arranged linearly along the direction orthogonal to the stretching/contracting direction, the stiffness of the stretchy sheet in the orthogonal direction will increase, and the flexibility of the sheet may decrease. Further, when a user sees such a stretchy sheet having creases extending continuously in the direction orthogonal to the stretching/contracting direction, the user may have an impression that the stretchy sheet is highly stretchable and may provide strong constriction.

The present invention is directed to provision of a stretchy sheet and an underpants-shaped absorbent article that have flexibility and tend to give an impression of a soft fitting.

### [Solution to Problem]

A main aspect of the present invention is a stretchy sheet having a vertical direction and a lateral direction intersecting each other, the stretchy sheet comprising: a pair of sheets; a plurality of welded portions joining the pair of sheets; a plurality of elastic members capable of stretching and contracting in the lateral direction, the plurality of elastic members being arranged with a space in the vertical direction and between the pair of sheets that are joined by the plurality of welded portions, the elastic members, in a state of contracting in the lateral direction, being each sandwiched between two welded portions adjacent to each other in the vertical direction, thereby restricting positions of the elastic members in the lateral direction with respect to the pair of sheets, the two welded portions included in the plurality of welded portions; and a plurality of welded-portion rows each having the plurality of welded portions that are arranged side-by-side in the vertical direction, the plurality of welded-portion rows including a first welded-portion row and a second welded-portion row that is located adjacent to the first welded-portion row on one side in the lateral direction, the first welded-portion row having a first convex portion and a second convex portion that protrude toward the one side in the lateral direction and that are adjacent to each other in the vertical direction, the second welded-portion row having a third convex portion that protrudes toward another side in the lateral direction, in a state where the stretchy sheet is stretched in the lateral direction, at least a part of a welded portion located furthest on the other side in the lateral direction in the third convex portion is located on the other side in the lateral direction with respect to a line connecting lateral other-side ends of welded portions located furthest on the one side in the lateral direction in the first convex portion and the second convex portion, the welded portion and the welded portions being included in the plurality of welded portions. Other features of the present disclosure will become apparent from the description in the present specification and the accompanying drawings.

### [Advantageous Effects of Invention]

According to the present invention, it is possible to provide a stretchy sheet and an underpants-shaped absorbent article that have flexibility and tend to give an impression of a soft fitting.

### [Brief Description of Drawings]

FIG. 1 is a schematic perspective view of a pull-on disposable diaper 1.
FIG. 2 is a schematic plan view of a diaper 1 in an unfolded and stretched state, as viewed from the skin surface side of a wearer.
FIG. 3 a schematic cross-sectional view taken along a line A-A in FIG. 2.
FIG. 4 is an illustrative diagram of welded-portion rows Ra, Rb provided to a front waist portion 20.
FIGS. 5A and 5B are illustrative diagrams showing a function of attaching a waist elastic member 23.
FIG. 6 is an illustrative diagram of welded-portion rows Ra in a first welded region A1.
FIG. 7 is an illustrative diagram of welded-portion rows Ra in a first welded region A1.
FIGS. 8A and FIG. 8B are illustrative diagrams of welded-portion rows Rb in a second welded region A2.
FIG. 9 is a schematic diagram showing creases 61, 62 formed in a front waist portion 20.
FIG. 10 is a photographic diagram illustrating creases 61 formed in a first stretchy region A1.

### [Description of Embodiments]

At least following matters will become apparent from the descriptions of the present specification and the accompanying drawings.

Aspect 1 is a stretchy sheet having a vertical direction and a lateral direction intersecting each other, the stretchy sheet comprising: a pair of sheets; a plurality of welded portions joining the pair of sheets; a plurality of elastic members capable of stretching and contracting in the lateral direction, the plurality of elastic members being arranged with a space in the vertical direction and between the pair of sheets that are joined by the plurality of welded portions, the elastic members, in a state of contracting in the lateral direction, being each sandwiched between two welded portions adjacent to each other in the vertical direction, thereby restricting positions of the elastic members in the lateral direction with respect to the pair of sheets, the two welded portions included in the plurality of welded portions; and a plurality of welded-portion rows each having the plurality of welded portions that are arranged side-by-side in the vertical direction, the plurality of welded-portion rows including a first welded-portion row and a second welded-portion row that is located adjacent to the first welded-portion row on one side in the lateral direction, the first welded-portion row having a first convex portion and a second convex portion that protrude toward the one side in the lateral direction and that are adjacent to each other in the vertical direction, the second welded-portion row having a third convex portion that protrudes toward another side in the lateral direction, in a state where the stretchy sheet is stretched in the lateral direction, at least a part of a welded portion located furthest on the other side in the lateral direction in the third convex portion is located on the other side in the lateral direction with respect to a line connecting lateral other-side ends of welded portions located furthest on the one side in the lateral direction in the first convex portion and the second convex portion, the welded portion and the welded portions being included in the plurality of welded portions.

According to Aspect 1, when the stretchy sheet contracts, arc-shaped creases along the convex portions are divided in the vertical direction, thereby being formed easily. Thus, the stretchy sheet is easily bent in the vertical direction, thereby being able to increase the flexibility of the stretchy sheet. In addition, in a state where the stretchy sheet is stretched, the first welded-portion row and the second welded-portion row are partially overlapped with each other in the lateral direction. Accordingly, the heights of the convex portions tend to be large and the gradients of the convex portions tend to be large. This makes it easier to form creases each having a large arc, and when a user sees such creases, the user is likely to have an impression that the stretchy sheet fits softly.

Aspect 2 is the stretchy sheet according to Aspect 1, wherein the plurality of welded-portion rows include a third welded-portion row that is located adjacent to the second welded-portion row on the one side in the lateral direction, the third welded-portion row has a fourth convex portion that protrudes toward the other side in the lateral direction, and in the state where the stretchy sheet is stretched in the lateral direction, a welded portion located furthest on the other side in the lateral direction in the fourth convex portion is located on the one side in the lateral direction with respect to the line, the welded portion being included in the plurality of welded portions.

According to Aspect 2, it is possible to prevent the welded-portion rows from being arranged too closely in the lateral direction. This can suppress reduction in the flexibility of the stretchy sheet in the lateral direction caused by the hardness of the welded portions. In addition, the area of the sheet that will form the arc-shaped creases between the first welded-portion row and the second welded-portion row can be secured, which makes it easier to form the arch-shaped creases.

Aspect 3 is the stretchy sheet according to Aspect 1 or 2, wherein in the state where the stretchy sheet is stretched in the lateral direction, a half or more of the welded portion in the lateral direction located furthest on the other side in the lateral direction in the third convex portion is located on the other side in the lateral direction with respect to the line.

According to Aspect 3, the length of an overlap in the lateral direction between the first welded-portion row and the second welded-portion row is elongated, and thus the height of the convex portion tends to be large, and the gradient of the convex portion tends to be large. This makes it easier to form large arc-shaped creases.

Aspect 4 is the stretchy sheet according to Aspect 3, wherein in the state where the stretchy sheet is stretched in the lateral direction, entirety of the welded portion located furthest on the other side in the lateral direction in the third convex portion is located on the other side in the lateral direction with respect to the line.

According to Aspect 4, the length of an overlap in the lateral direction between the first welded-portion row and the second welded-portion row is elongated, and thus the height of the convex portion tends to be large, and the gradient of the convex portion tends to be large. This makes it easier to form large arc-shaped creases.

Aspect 5 is the stretchy sheet according to any of Aspects 1 to 4, wherein in the state where the stretchy sheet is stretched in the lateral direction, a lateral interval between the first welded-portion row and the second welded-portion row is equal to or more than 6 mm.

According to Aspect 5, the welded-portion rows are prevented from being arranged closely in the lateral direction, as compared to the case where the lateral interval between the first welded-portion row and the second welded-portion row is less than 6 mm. Accordingly, it is possible to suppress reduction in the flexibility of the stretchy sheet in the lateral direction caused by the hardness of the welded portions. Further, the area of the sheet that will form arc-shaped creases between the first welded-portion row and the second welded-portion row can be secured, which makes it easier to form the arc-shaped creases.

Aspect 6 is the stretchy sheet according to any of Aspects 1 to 5, wherein the first convex portion is located on an upper side in the vertical direction with respect to the second convex portion, the stretchy sheet has, in the state of being stretched in the lateral direction, another line connecting lateral other-side ends of the plurality of welded portions constituting the second welded-portion row, a first intersection at which the other line and the line intersect each other on the upper side, and a second intersection at which the other line and the line intersect each other on a lower side in the vertical direction, and a vertical length from the lateral other-side end of the welded portion located furthest on the one side in the lateral direction in the first convex portion to the first intersection is longer than a vertical length from the first intersection to the second intersection.

According to Aspect 6, the gradient of the third convex portion tends to be large, and accordingly the arc-shaped creases with large gradients are easily formed. Thus, the user is likely to recognize the arc-shaped creases.

Aspect 7 is the stretchy sheet according to any of Aspects 1 to 6, wherein the plurality of elastic members include a first elastic member and a second elastic member that is located adjacent to the first elastic member on a lower side in the vertical direction, and a welded portion on the lower side of the two welded portions sandwiching the first elastic member in the vertical direction is non-continuous with a welded portion on an upper side of the two welded portions sandwiching the second elastic member in the vertical direction.

According to Aspect 7, it is possible to suppress an increase in the stiffness in the vertical direction of the stretchy sheet caused by the welded portions that are continuous in the vertical direction, thereby being able to increase the flexibility of the stretchy sheet in the vertical direction.

Aspect 8 is an underpants-shaped absorbent article having the stretchy sheet according to any of Aspects 1 to 7, the underpants-shaped absorbent article having a vertical direction and a lateral direction intersecting each other, the underpants-shaped absorbent article comprising an absorbent main body; and a pair of waist portions, at least one of the pair of waist portions including the stretchy sheet, the vertical direction of the stretchy sheet being aligned with the vertical direction of the underpants-shaped absorbent article, the lateral direction of the stretchy sheet being aligned with the lateral direction of the underpants-shaped absorbent article.

According to Aspect 8, the arch-shaped creases along the convex portions are divided in the vertical direction to be formed in the waist portion (s), and thus the waist portion(s) can be easily bent in the vertical direction, which increases the flexibility of the waist portion(s). Further, when the user sees the arc-shaped creases, the user is likely to have an impression that the waist portion(s) fit softly.

Aspect 9 is the underpants-shaped absorbent article according to Aspect 8, wherein the stretchy sheet includes a first welded region, the first welded region including a plurality of combinations of the first welded-portion row and the second welded-portion row that are adjacent to each other in the lateral direction.

According to Aspect 9, multiple arc-shaped creases are easily formed in the lateral direction in the waist portion(s), and accordingly the user is likely to have an impression that the waist portion(s) fit softly.

Aspect 10 is the underpants-shaped absorbent article according to Aspect 9, wherein the pair of waist portions are joined, at lateral side portions thereof, by a pair of side joining portions, and the first welded region is overlapped, in the lateral direction, with at least a part of one of the pair of the side joining portions on the one side in the lateral direction, and overlapped, in the lateral direction, with at least a part of another of the pair of the side joining portions on the other side in the lateral direction.

According to Aspect 10, multiple arch-shaped creases are likely to be formed in the lateral direction over a wide range in the lateral direction of the waist portion(s), and accordingly the user is likely to have an impression that the waist portion(s) fit softly.

Aspect 11 is the underpants-shaped absorbent article according to Aspect 9 or 10, wherein the stretchy sheet further includes a second welded region, in the second welded region, a plurality of fourth welded-portion rows are arranged in the lateral direction, the plurality of fourth welded-portion rows each having convex portions that protrude alternately toward the one side and the other side in the lateral direction, in a state where the underpants-shaped absorbent article is stretched in the lateral direction, the fourth welded-portion rows adjacent to each other in the lateral direction are not overlapped with each other in the lateral direction or a length of an overlap therebetween in the lateral direction is less than or equal to a lateral length of a welded portion located furthest on a protruding side in the lateral direction in the convex portions, the welded portion being included in the plurality of welded portions, a lateral interval between the fourth welded-portion rows is smaller than a lateral interval between the first welded-portion row and the second welded-portion row, the second welded region is located in an upper end portion of the waist portion with a predetermined width, and the first welded region is located on a lower side with respect to the second welded region.

According to Aspect 11, in the second welded region, creases closer to creases extending along the vertical direction are formed at intervals smaller than the intervals thereof in the first welded region. Thus, the user is likely to have an impression of the waist portion(s) that the upper portion (second welded region A2) tightly fits and is less likely to slip downward, while other part (first welded region A1) fits softly and is comfortable.

### Embodiment

Hereinafter, an embodiment will be described using an example of a pull-on disposable diaper for adults as an underpants-shaped absorbent article using a stretchy sheet according to a present embodiment. However, the underpants-shaped absorbent article is not limited to the above, and may be, for example, a pull-on disposable diaper for infants, a shorts-type sanitary napkin, or the like.

### Basic configuration of pull-on disposable diaper 1

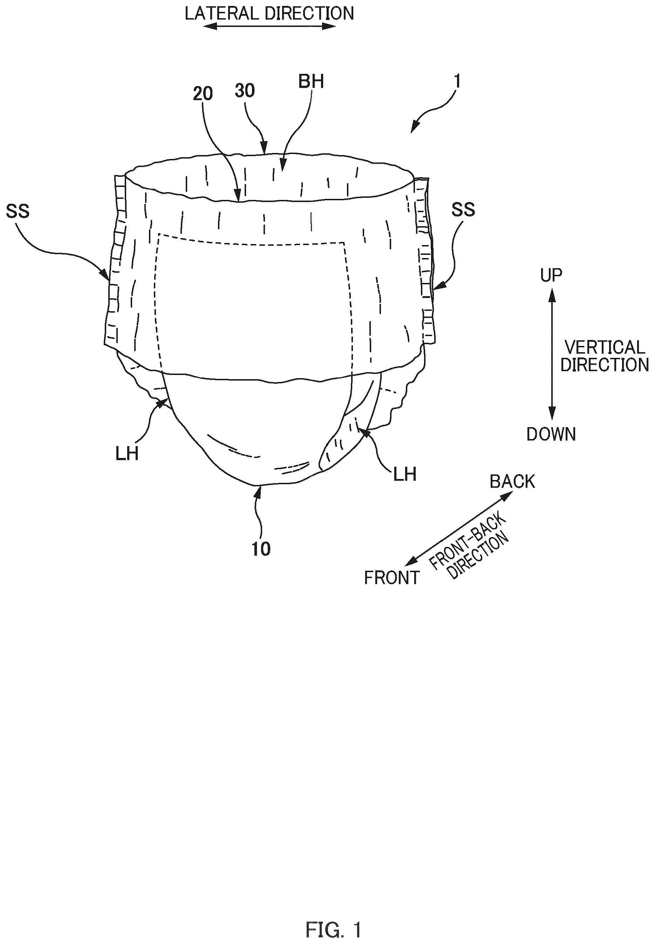

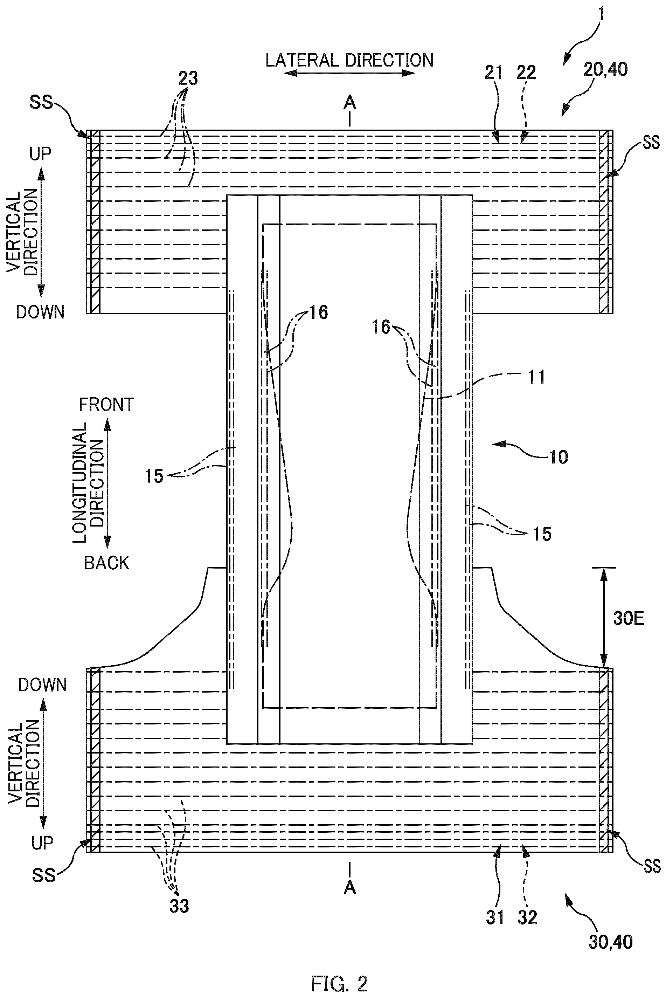

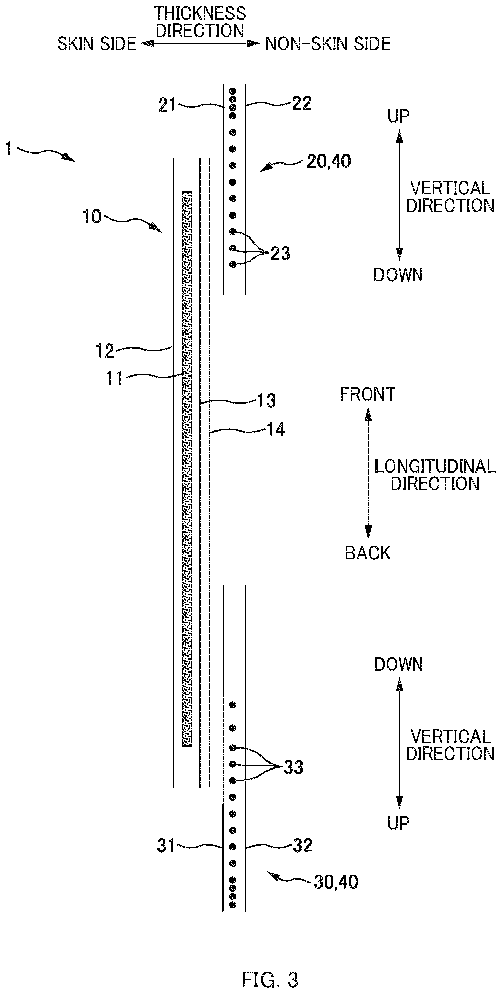

FIG. 1 is a schematic perspective view of a pull-on disposable diaper 1 (hereinafter, also referred to as "diaper"). FIG. 2 is a schematic plan view of the diaper 1 in an unfolded and stretched state, as viewed from the skin surface side of a wearer. FIG. 3 a schematic cross-sectional view thereof taken along a line A-A in FIG. 2.

The diaper 1 has a vertical direction, a lateral direction, and a front-back direction that are perpendicular to one another, and has a waist opening BH and a pair of leg openings LH. In the vertical direction, the side corresponding to the waist opening BH is the upper side, and the side corresponding to the wearer's crotch is the lower side. In the front-back direction, the side corresponding to the wearer's stomach is the front side, and the side corresponding to the wearer's back is the back side. In addition, the diaper 1 has a thickness direction in which materials are layered as shown in FIG. 3, and in the thickness direction, the side that comes into contact with the wearer will be referred to as skin side, and the side opposite thereto will be referred to as non-skin side.

The diaper 1 includes an absorbent main body 10 and a pair of waist portions 20, 30. The pair of waist portions 20, 30 is joined to the non-skin-side surface of the absorbent main body 10 with an adhesive or the like. The one on the front side out of the pair of waist portions 20 is also referred to as front waist portion 20, and the one on the back side is also referred to as back waist portion 30. The front waist portion 20 and the back waist portion 30 are joined by a pair of side joining portions SS at lateral side portions. Examples of the joining method by the side joining portions SS include thermal welding, ultrasonic welding, and joining with an adhesive.

As shown in FIG. 3, the absorbent main body 10 has an absorbent core 11, a liquid-permeable top sheet 12 arranged on the skin side with respect to the absorbent core 11, a liquid-impermeable back sheet 13 arranged on the non-skin side with respect to the absorbent core 11, and an exterior sheet 14. The absorbent core 11 may be any material that absorbs and retains excreted fluid, and examples thereof include those formed by molding liquid absorbent material such as pulp fibers and superabsorbent polymer into a predetermined shape. The absorbent core 11 may be covered with a liquid permeable sheet such as tissue paper, nonwoven fabric, or the like.

As shown in FIG. 2, in the two lateral sides of the absorbent main body 10, leg elastic members 15 (e.g., elastic strings) may be provided, which stretches and contracts along a longitudinal direction of the absorbent main body 10. Furthermore, on the inner side in the lateral direction with respect to the leg elastic members 15, leak-proof wall portions capable of standing up toward the skin side may be provided. The leak-proof wall portions are formed as follows, for example, the two lateral side portions of the exterior sheet 14 are folded inward in the lateral direction so as to be located on the skin-side surface of the top sheet 12, and the leak-proof wall elastic members 16 (e.g., elastic strings) that stretch and contract in the longitudinal direction are provided in the folded portion.

In the present embodiment, portions overlapped with the side joining portions SS in the vertical direction correspond to the waist portions 20, 30, and the front waist portion 20 and the back waist portion 30 are portions that are rectangular in a plan view. The back waist portion 30 has a substantially trapezoidal shaped extension portion 30E on the crotch side thereof. The extension portion 30E can cover the wearer's buttocks portion. In the following description, the back waist portion 30 and the extension portion 30E are also collectively referred to as back exterior portions 30, 30E. However, there is no limitation to the above and, for example, it may have a configuration without the extension portion 30E on the back side, or may have a configuration including a portion extending from the front waist portion 20 toward the crotch side.

The front waist portion 20 and the back exterior portions 30, 30E each have skin-side sheets 21, 31, non-skin side sheets 22, 32, and waist elastic members 23, 33 (e.g., elastic strings) that can stretch and contract in the lateral direction. The waist elastic members 23, 33 are arranged side by side with a space in the vertical direction, between the skin-side sheet 21, 31 and the non-skin side sheet 22, 32.

It is preferable that the skin-side sheets 21, 31 and the non-skin side sheets 22, 32 are each a flexible sheet member, and examples thereof includes spunbond nonwoven fabric, meltblown nonwoven fabric, SMS (spunbond/meltblown/spunbond) nonwoven fabric, and the like. The constituent fibers of the nonwoven fabric are not limited to mono fibers made of polypropylene (PP), which is a typical example of thermoplastic resin, but mono fibers made of another thermoplastic resin such as polyethylene (PE) may be used, and further composite fibers having a core-sheath structure including PE, PP, and the like may be used.

The basic configuration of the diaper 1 has been described above, but the above configuration of the diaper 1 is merely an example and is not intended to be limited thereto. For example, in the diaper 1 of the present embodiment, the front waist portion 20 and the back exterior portions 30, 30E are constituted by separate members, and the non-skin-side surface of the absorbent main body 10 is exposed in the crotch portion. There is no limitation thereto and, for example, the diaper may have an exterior member constituted by three components: the front waist portion 20, the back exterior portions 30, 30E, and the crotch portion connecting them, or the diaper may have an exterior member constituted by a single component that extends continuously from the front waist portion 20 to the back waist portion 30.

### Waist portions 20, 30 (stretchy sheet 40)

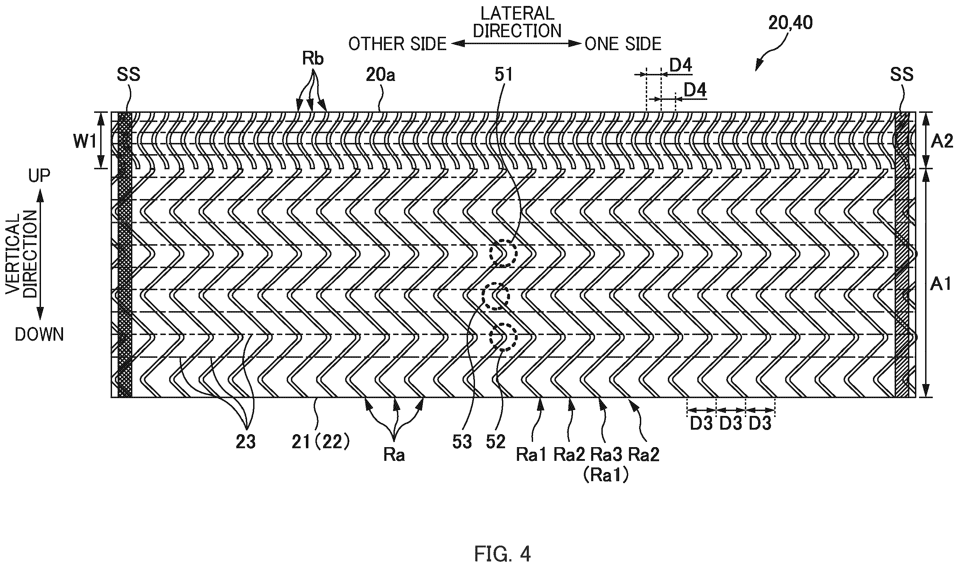

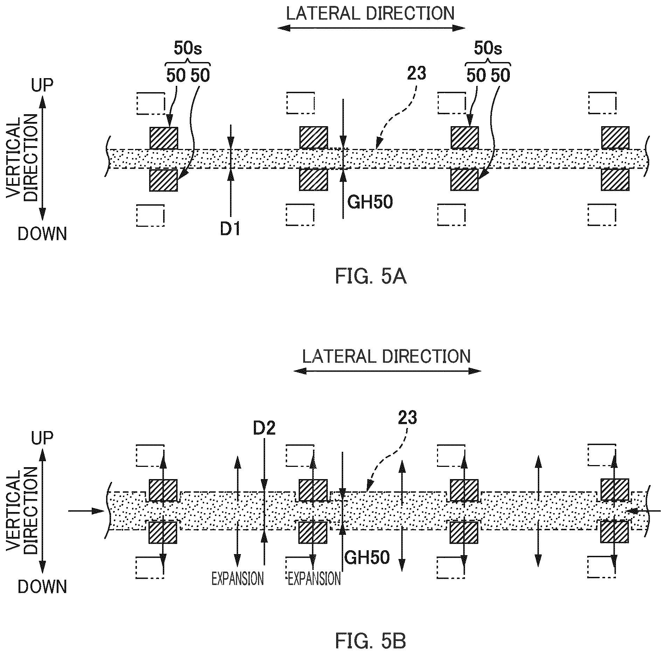

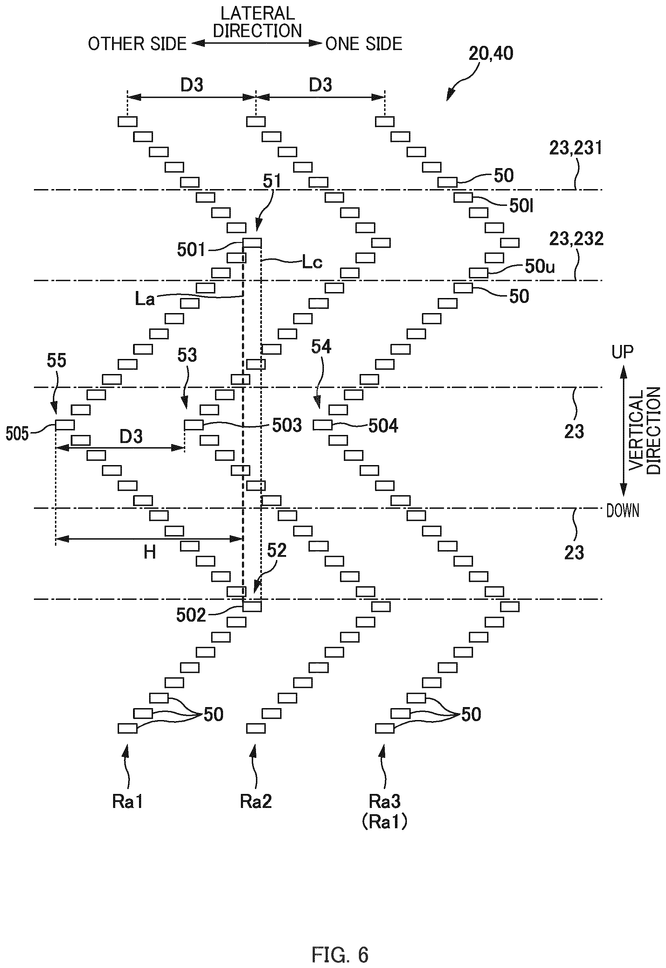

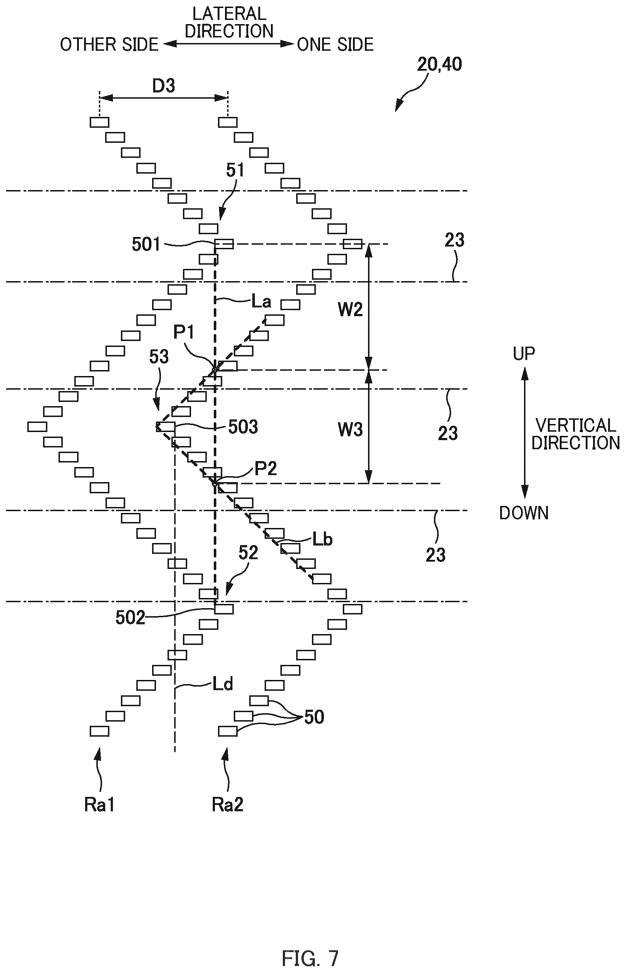

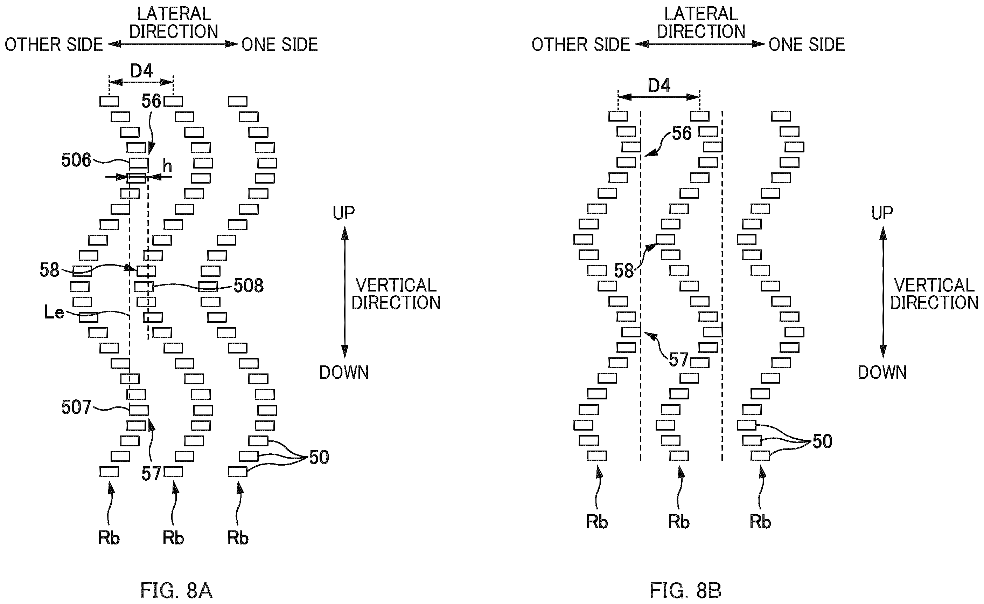

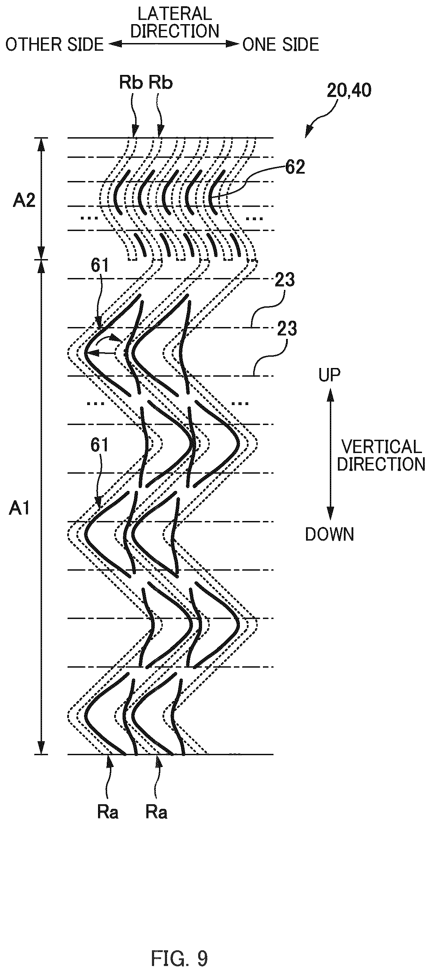

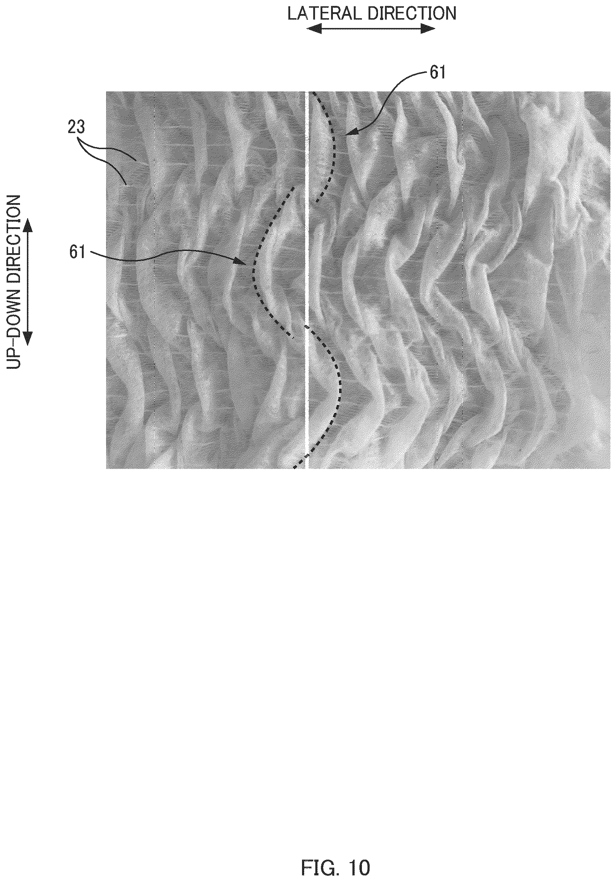

FIG. 4 is an illustrative diagram of welded-portion rows Ra, Rb provided in the front waist portion 20. FIGS. 5A and 5B are illustrative diagrams showing a function of attaching the waist elastic member 23. FIGS. 6 and 7 are illustrative diagrams of the welded-portion rows Ra included in a first welded region A1. FIGS. 8A and 8B are illustrative diagrams of the welded-portion rows Rb included in a second welded region A2. FIG. 9 is a schematic diagram showing creases 61, 62 formed in the front waist portion 20. FIG. 10 is a photographic diagram illustrating the creases 61 formed in the first stretchy region A1.

FIGS. 4, 6, 7, 8A, and 8B show a state where the front waist portion 20 (the stretchy sheet 40) stretches in the lateral direction. The state where the waist portions 20, 30 and/or the stretchy sheet 40 stretches in the lateral direction refers to the state where they are stretched to the extent that creases formed in the waist portions 20, 30 and/or the stretchy sheet 40 can substantially no longer be seen, and the dimensions of the members constituting the waist portions 20, 30 and/or the stretchy sheet 40 (e.g., the skin-side sheet 21, etc.) match or are close to the dimensions of the members of their own (i.e., the dimensions when stretchability of the elastic members is not exhibited).

### Position restriction of waist elastic members 23

The front waist portion 20 and the back waist portion 30 are constituted by the stretchy sheet 40 in which the waist elastic members 23, 33 (elastic members) that can stretch and contract in the lateral direction are arranged side-by-side with a space in the vertical direction, between the skin-side sheets 21, 31 and the non-skin side sheets 22, 32 (between a pair of sheets) that are joined by the welded portions 50. As shown in FIG. 6, the skin-side sheets 21, 31 and the non-skin side sheets 22, 32 are joined to each other by the welded portions 50 that are arranged discretely in the vertical direction and the lateral direction. Examples of the method of forming the welded portions 50 can include a known method such as ultrasonic welding and heat welding.

A pair of sheets that are joined by the welded portions 50 may be two separate sheets such as the skin-side sheets 21, 31 and the non-skin side sheets 22, 32 in the present embodiment, or may be a sheet having two layers obtained by folding back a single sheet. Further, the welded portions 50 are not limited to joining only the pair of sheets sandwiching the waist elastic members 23, 33, but may join sheets with three or more layers including sheet overlaid on the pair of sheets.

Since the front waist portion 20 and the back waist portion 30 have substantially the same configuration, the following describes the front waist portion 20 as a representative of both. The front waist portion 20 (the stretchy sheet 40) has the multiple welded portion rows Ra, Rb in which the multiple welded portions 50 are arranged in the vertical direction. The multiple welded-portion rows Ra, Rb are arranged at intervals D3, D4 in the lateral direction. As shown in FIG. 4, the welded-portion rows Ra, Rb in the present embodiment extend in the vertical direction while meandering in the lateral direction. In other words, as shown in FIG. 6, the positions of the welded portions 50 adjacent in the vertical direction are shifted in the lateral direction.

Meanwhile, the positions in the vertical direction of the welded portions 50 adjacent in the lateral direction are aligned. Thus, as shown in FIG. 5A, the positions of the spaces through which the waist elastic members 23 extend between the welded portions 50 adjacent to each other in the vertical direction are aligned in the vertical direction, so that the waist elastic members 23 can be arranged along the lateral direction. However, there is no limitation to this configuration, and the positions of the welded portions 50 adjacent to each other in the lateral direction may be shifted in the vertical direction, or the waist elastic members 23 may extend between the welded portions 50 whose positions are different in the vertical direction. In these cases as well, the front waist portion 20 can also be given stretchability in the lateral direction.

As shown in FIG. 5A, two welded portions 50 located on two sides of the waist elastic member 23 in the vertical direction are also referred to as "welded portion pair 50S". An upper welded portion 50 and a lower welded portion 50 that form the welded portion pair 50S are aligned in the vertical direction with a space GH50 therebetween. The size of the space GH50 is set to be equal to or slightly larger than an outer diameter D1 of the waist elastic member 23 when the front waist portion 20 is stretched to the maximum lateral length (GH50 ≥ D1).

However, the waist elastic member 23 becomes thicker as it contracts in the lateral direction. Thus, as shown in FIG. 5B, the space GH50 of the welded portion pair 50S is made smaller than the maximum outer shape D2 of the waist elastic member 23 when the waist elastic member 23 contracts in the lateral direction (i.e., when the front waist portion 20 is in a natural state) (GH50 < D2). According to this configuration, the waist elastic member 23 that is contracted in the lateral direction is sandwiched in the vertical direction between the welded portion pair 50S (expansion in the vertical direction is restricted). Thus, the position (movement) of the waist elastic member 23 in the lateral direction and the vertical direction is restricted with respect to the skin side sheet 21 and the non-skin side sheet 22.

Accordingly, even without fixing the waist elastic member 23 to the skin side sheet 21 and the non-skin side sheet 22 using an adhesive, it is possible to suppress positional shifting of the waist elastic member 23 and maintain stretchability of the front waist portion. In other words, with the use of the welded portions 50, it is possible to attach the waist elastic member 23 to the skin-side sheet 21 and the non-skin side sheet 22 without using an adhesive or with a reduced amount of an adhesive used. This makes it possible to suppress a reduction in the flexibility of the front waist portion 20 (the stretchy sheet 40) caused by hardening of the adhesive. In addition, it is also possible to suppress the deterioration of the elasticity of the waist elastic members 23 caused by hardening of the adhesive.

Further, in the front waist portion 20, the waist elastic member 23 is fixed to the skin-side sheet 21 and the non-skin side sheet at the side joining portions SS. Specifically, when the diaper 1 is manufactured, the side joining portions SS are formed in the state where the waist elastic member 23 in a stretched state is sandwiched between the skin-side sheet 21 and the non-skin side sheet 22, and the end portions of the waist elastic member 23 are fixed to the side joining portions SS. Thus, even if the front waist portion 20 stretches significantly when the diaper 1 is putting on, it is possible to prevent the waist elastic member 23 from coming off from between the welded portion pair 50S, thereby being able to maintain the elasticity of the front waist portion 20.

Further, the relationship between the space GH50 between the welded portion pair 50S and the outer diameters D1, D2 of the waist elastic member 23 is not limited to the above. For example, the waist elastic member 23 may be partially overlapped with the welded portion 50 to such an extent that the waist elastic member 23 does not break. In this case as well, the waist elastic member 23, in a state of being contracted in the lateral direction, is sandwiched between the welded portion pair 50S, and the position of the waist elastic member 23 in the lateral direction and the vertical direction is restricted with respect to the skin side sheet 21 and the non-skin side sheet 22.

Furthermore, it is not necessarily the case that all of the waist elastic members 23 (elastic members) arranged in the front waist portion 20 (the stretchy sheet 40) are each sandwiched between the welded portion pair 51 to restrict their positions. It is sufficient that the position(s) of at least a part of the waist elastic members 23 is restricted by the welded portion pair(s) 51, and another part of the waist elastic members 23 may be fixed to the skin side sheet 21 and the non-skin side sheet 22 with an adhesive.

### First welded region A1

As shown in FIG. 4, the front waist portion 20 has the first welded region A1 and the second welded region A2, which have welded-portion rows Ra, Rb with different shapes and spaces therebetween. First, the first welded region A1 will be described. The first welded region A1 is located on the lower side with respect to the second welded region A2 and is longer in the vertical direction than the second welded region A2. In the first welded region A1, the welded-portion rows Ra having the same arrangement pattern of the welded portions 50 are arranged side-by-side in the lateral direction at equal intervals D3. The welded-portion rows Ra each extend in the vertical direction while meandering in the lateral direction, and have a shape having convex portions arranged in the vertical direction, the convex portions protruding toward one side and the other side in the lateral direction alternately.

In the present embodiment, the welded portions 50 have a rectangular shape whose length is longer in the lateral direction, but the shape of the welded portions 50 is not particularly limited. Further, in the present embodiment, all the welded portions 50 that belong to the welded-portion rows Ra have the same shape, but there is no limitation to this configuration, and they may have a configuration including different shapes. Furthermore, the welded-portion rows Ra, Rb illustrated in FIG. 4 extend along the vertical direction, but there is no limitation to this configuration, and the welded-portion rows each may extend in a direction inclined with respect to the vertical direction such that the upper end thereof is shifted in the lateral direction from the lower end thereof.

When the front waist portion 20 contracts in the lateral direction, the welded portions 50, which have high stiffness, are less likely to contract, and thus the interval between the welded-portion rows Ra adjacent to each other in the lateral direction decreases. Then, portions of the skin-side sheet 21 and the non-skin side sheet 22 that are located between the welded-portion rows Ra adjacent to each other in the lateral direction bulge in such a manner as to protrude outward in the thickness direction, so that creases are likely to be formed along the shape of the welded-portion rows Ra.

Here suppose that welded-portion rows that are different from the welded-portion rows Ra in the first welded region A1 are formed, for example, such that the welded-portion rows extend linearly in the vertical direction, in other words, the welded portions at the same lateral position are lined up in the vertical direction. In this case, long straight creases along the vertical direction are more likely to be formed. Such creases may act like a tension rod, thereby increasing the stiffness in the vertical direction of the front waist portion, which may decrease the flexibility of the front waist portion. In addition, the welded portion pairs each sandwiching the waist elastic member are concentrated at the same position in the lateral direction, and thus the waist elastic member is more likely to adhere locally to a single part of the wearer, which may cause a gather mark to be left on the skin or degradation of sense of comfort.

In contrast, in the first welded region A1, the welded portion rows Ra each meandering in the lateral direction are formed. Accordingly, curved creases along the convex portions of the welded-portion rows Ra are formed, with the creases being divided in the vertical direction while their directions of curves in the lateral direction being changed. Thus, the first welded region A1 is easily bent in the vertical direction, which increases the flexibility in the vertical direction.

Further, when focusing on a portion of the first welded region A1, the first welded region A1 includes a first welded-portion row Ra1 and a second welded-portion row Ra2 located adjacent to the first welded-portion row Ra1 on the one side in the lateral direction. As shown in FIG. 6, the first welded-portion row Ra1 has a first convex portion 51 and a second convex portion 52 that protrude toward the one side in the lateral direction (the side of the second welded-portion row Ra2) and that are adjacent to each other in the vertical direction. The second welded-portion row Ra2 has a third convex portion 53 that protrudes toward the other side in the lateral direction (the side of the first welded-portion row Ra1). The third convex portion 53 is located between the first convex portion 51 and the second convex portion 52 in the vertical direction.

In a state where the front waist portion 20 is stretched in the lateral direction, a line connecting the lateral other-side ends of the welded portions 501, 502 that are located furthest on the one side in the lateral direction in the first convex portion 51 and the second convex portion 52, respectively, is defined as a virtual line La. In this event, at least a part of a welded portion 503 located furthest on the other side in the lateral direction in the third convex portion 53 of the second welded-portion row Ra2 is located on the other side in the lateral direction with respect to the virtual line La.

In other words, the virtual line La corresponds to the base of the convex portion (hereinafter, a fifth convex portion 55) of the first welded-portion row Ra1 that protrudes toward the other side in the lateral direction, and the third convex portion 53 extends to the inner side (the other side in the lateral direction) with respect to the virtual line La. In other words, the height H (length in the lateral direction) of the fifth convex portion 55 is larger than the lateral interval D3 between the first welded-portion row Ra1 and the second welded-portion row Ra2 (H > D3) so that the first welded-portion row Ra1 and the second welded-portion row Ra2 are partially overlapped in the lateral direction.

Thus, when the front waist portion 20 contracts, the fifth convex portion 55 and the third convex portion 53 are more likely to come closer to each other, as compared to the case where the third convex portion 53 is located on the outer side (the one side of the lateral direction) with respect to the virtual line La. Therefore, an unwelded sheet portion between the fifth convex portion 55 and the third convex portion 53 bulges outward in the thickness direction along the fifth convex portion 55 and the third convex portion 53. The outwardly bulging sheet portion is likely to collapse toward the base side of the fifth convex portion 55 (the one side in the lateral direction), and as a result, as shown in FIGS. 9 and 10, arc-shaped (curved line-shaped) creases 61 are likely to be formed along the convex portions of the welded-portion rows Ra.

Further, as described above, the height H of the fifth convex portion 55 is larger than the lateral interval D3 between the welded-portion rows Ra, which means that the amount of meandering in the lateral direction of the fifth convex portion 55 is large, and the gradient of the fifth convex portion 55 tends to be large (the slope tends to be steeper). Thus, the larger arc-shaped creases 61 are more likely to be formed. Accordingly, a user is likely to recognize that arc-shaped creases 61 are formed, which are different from typical creases formed in a waist portion such as vertical creases along the vertical direction formed when waist elastic members are fixed to a sheet with an adhesive.

Further, a user who sees the arc-shaped creases 61 is more likely to have an impression of flexible fitting of the front waist portion 20 with a constricting feeling being mitigated, as compared to the case where the user sees typical vertical creases formed in the waist portion. In fact, as compared to the vertical creases that are firmly pushed up in the thickness direction by the welded-portion rows along the vertical direction, the arc-shaped creases 61 that are pushed up while bending in the lateral direction along the shape of the convex portions of the welded-portion rows Ra tend to provide a flexible feeling.

Further, as described above, the front waist portion 20 of the present embodiment has high flexibility, since the waist elastic members 23 are attached without using an adhesive (or with a reduced amount of adhesive). Therefore, the user who sees the arc-shaped creases 61 and have an impression that the front waist portion 20 is flexible can be more likely to feel the flexibility of the front waist portion 20 by virtue of an adhesive not being used. Furthermore, with an increase in the flexibility of the front waist portion 20, the front waist portion 20 is more likely to fit along the wearer's body.

Note that FIG. 10 is a photograph of a stretchy sheet in the natural state that was actually produced such that elastic members are arranged between a pair of sheets, to explain the arc-shaped creases 61. Specifically, the lateral length of the welded portions 50 was set to 2.5 mm, the intervals (D3) between welded portion rows Ra was set to 15 mm, the height (H) of the convex portions was set to 25.6 mm, and the length of the base of the convex portions (the vertical length from the welded portion 501 to the welded portion 502) was set to 50 mm.

Further, as described above, it is sufficient that at least a part of the welded portion 503 located furthest on the other side in the lateral direction in the third convex portion 53 is located on the inner side (on the other side in the lateral direction) with respect to the virtual line La. However, preferably, a half or of the welded portion 503 in the lateral direction is located on the inner side (on the other side in the lateral direction) with respect to the virtual line La. More preferably, as shown in FIG. 6, the entirety of the welded portion 503 is located on the other side in the lateral direction with respect to the virtual line La.

According to this configuration, the overlapping length between the first welded-portion row Ra1 and the second welded-portion row Ra2 is elongated in the lateral direction. Thus, when the front waist portion 20 contracts, the fifth convex portion 55 and the third convex portion 53 are likely to come close each other, and arc-shaped creases 61 along the convex portions are likely to be formed. Further, the overlapping length between the first welded-portion row Ra1 and the second welded-portion row Ra2 in the lateral direction being long means that the height H of the convex portion of the welded-portion row Ra tends to be large and the gradient of the convex portion tends to be large. Accordingly, the larger arc-shaped creases 61 are likely to be formed, and thus the user is likely to have an impression that the front waist portion 20 is flexible.

As shown in FIG. 6, the first welded region A1 also includes a third welded-portion row Ra3 that is located adjacent to the second welded-portion row Ra2 on the one side in the lateral direction. The third welded-portion row Ra3 has a fourth convex portion 54 that protrudes toward the other side in the lateral direction. The fourth convex portion 54 is located in the same position in the vertical direction as the third convex portion 53, and is located between the first convex portion 51 and the second convex portion 52 in the vertical direction.

Then, in the state where the front waist portion 20 is stretched in the lateral direction, it is preferable that a welded portion 504 located furthest on the other side (second welded-portion row Ra2 side) in the lateral direction in the fourth convex portion 54 is located on the outer side (the one side in the lateral direction) with respect to the virtual line La. In other words, it is preferable that the height H of the fifth convex portion 55 in the first welded-portion row Ra1 is shorter than the length corresponding to twice the lateral interval D3 between the welded-portion rows Ra (H < 2×D3).

In other words, the first welded-portion row Ra1 is overlapped with the second welded-portion row Ra2 that is adjacent thereto in the lateral direction, but is not overlapped with the third welded-portion row Ra3 that is adjacent thereto next, or is overlapped therewith only slightly (less than the lateral length of the welded portions 50).

According to this configuration, the lateral interval D3 of the welded-portion rows Ra is set to be relatively large, thereby being able to prevent the welded-portion rows Ra from being arranged closely in the lateral direction. Thus, it is possible to suppress reduction in the flexibility of the front waist portion 20 in the lateral direction caused by the hardness of the welded portions 50.

Further, the lateral interval D3 of the welded-portion rows Ra being appropriately increased ensures the area of the sheet between the welded-portion rows Ra, in other words, the area of the sheet that will bulge outward in the thickness direction when the front waist portion 20 contracts. Accordingly, the arc-shaped creases 61 elongated in the vertical direction are likely to be formed. However, there is no limitation to the above configuration, the fourth convex portion 54 (the welded portion 504) of the third welded-portion row Ra3 may be located on the inner side (the other side in the lateral direction) with respect to the virtual line La.

Further, in the state where the front waist portion 20 is stretched in the lateral direction, the lateral interval D3 between the first welded-portion row Ra1 and the second welded-portion row Ra2 is preferably 6 mm or larger. According to this configuration, it is possible to prevent the welded-portion rows Ra from being arranged closely in the lateral direction, as compared with the case where the lateral interval D3 between the welded-portion rows Ra is less than 6 mm. Accordingly, it is possible to suppress reduction in the flexibility of the front waist portion 20 caused by the hardness of the welded portions 50.

Further, the first welded-portion row Ra1 and the second welded-portion row Ra2 are partially overlapped in the lateral direction (the relationship of H > D3 is established) although the lateral interval D3 between the welded-portion rows Ra is set to be 6 mm or more such that they are appropriately separated. Thus it can be said that the height H of the welded-portion rows Ra tends to be large, and the gradient thereof tends to large. Accordingly, the larger arc-shaped creases 61 are likely to be formed, and thus the user is likely to have an impression that the front waist portion 20 is flexible. However, there is no limitation to the above configuration, and the interval D3 may be less than 6 mm.

Note that as shown in FIG. 6, the interval D3 is defined as the lateral length between the lateral center of one of the welded portions 50 belonging to the first welded-portion row Ra1 and the lateral center of the welded portion 50 that belongs to the second welded-portion row Ra2 and that is located at the position being the same in the vertical direction as or being closest in the vertical direction to the position of the one of the welded portions 50 in the first welded-portion row Ra1.

In addition, the height H of the convex portion included in the welded-portion row Ra is defined as the lateral length between the welded portion (e.g., the welded portion 501) located furthest on the one side in the convex portion (e.g, the first convex portion 51) that protrudes toward the one side in the lateral direction, and the welded portion (e.g, the welded portion 505) located furthest on the other side in the convex portion (e.g., the fifth convex portion 55) that is adjacent in the vertical direction to the above convex portion and that protrudes toward the other side in the lateral direction.

Further, as shown in FIG. 7, in the first welded-portion row Ra, the first convex portion 51 is located on the upper side in the vertical direction with respect to the second convex portion 52. In the state where the front waist portion 20 is stretched in the lateral direction, the line (another line) connecting the lateral other-side ends of the multiple welded portions 50 constituting the second welded-portion row Ra2 is defined as a virtual line Lb. Note that as shown in FIG. 7, when the welded portions 50 each have a rectangular shape or the like and have a lateral other-side end being a side with a vertical length, the line connecting the vertical centers of the sides is defined as the virtual line Lb. Further, the point at which the virtual line Lb and the above-described virtual line La intersect on the upper side in the vertical direction is defined as a first intersection P1, and the point at which the virtual line Lb and the virtual line La intersect on the lower side in the vertical direction is defined as a second intersection P2.

In this event, it is preferable that the vertical length W2 from the lateral other-side end of the welded portion 501 that is located furthest on the one side in the lateral direction in the first convex portion 51 to the first intersection P1 is longer than the vertical length W3 from the first intersection P1 to the second intersection P2 (W2 > W3). Note that when the lateral other-side end of the welded portions 501 has a side with a vertical length, W2 is a length from the vertical center of that side.

The length W3 in the vertical direction from the first intersection P1 to the second intersection P2 being relatively short means that the gradient of the third convex portion 53 tends to be large. Thus, the creases 61 having a shape of an arc with a large gradient are easily formed along the convex portions of the welded-portion row Ra, and the arc-shaped creases 61 are easily recognized by the user. Further, it can also be said that the third convex portion 53 does not extend too far to the inner side (the other side in the lateral direction) with respect to the virtual line La. This prevents the welded-portion rows Ra from being arranged side-by-side closely in the lateral direction, thereby ensuring the flexibility of the front waist portion 20. In addition, the area of the sheet that will bulge outward in the thickness direction when the front waist portion 20 contracts is secured, which makes it easier to form arc-shaped creases 61. However, there is no limitation to the above configuration, and the length W2 may be equal to or less than the length W3.

In addition, with the welded-portion row Ra having a convex portion, the arc-shaped creases 61 extending along the convex portion is more easily formed. However, if the convex portion of the welded-portion row Ra is formed with welded portions that are continuous in the vertical direction, the hardness of the welded portions will increase the stiffness of the front waist portion 20 in the vertical direction. Thus, as shown in FIG. 6, it is preferable that the welded portions 50 are arranged at intervals in the vertical direction, while the positions of the welded portions 50 adjacent in the vertical direction are shifted in the lateral direction such that the welded-portion row Ra has a convex shape.

Specifically, as shown in FIG. 6, of two welded portions 50 that sandwich a certain waist elastic member 231 (first elastic member) in the vertical direction, a lower welded portion 50l is non-continuous with an upper welded portion 50u of another two welded portions 50 that sandwich, in the vertical direction, a waist elastic member 232 (second elastic member) located adjacent to and below the waist elastic member 231. This configuration can prevent an increase in the stiffness in the vertical direction of the front waist portion 20 caused by the hardness of the welded portions 50, which facilitates bending of the front waist portion 20 in the vertical direction, thereby being able to increase the flexibility of the front waist portion 20 in the vertical direction.

Further, in the front waist portion 20, the multiple waist elastic members 23 are arranged in the vertical direction, but the number and interval thereof are not particularly limited. However, it is preferable that at least one waist elastic member 23 is provided between two convex portions that protrude toward the same side in the lateral direction and that are adjacent to each other in the vertical direction, in the welded-portion row Ra, such as between the welded portion 501 of the first convex portion 51 and the welded portion 502 of the second convex portion 52. According to this configuration, when the front waist portion 20 contracts, the welded portion rows Ra adjacent to each other in the lateral direction easily come close to each other (the fifth convex portion 55 and the third convex portion 53 easily come close to each other) between two convex portions (e.g., the first convex portion 51 and the second convex portion 52) adjacent in the vertical direction, which makes it easier to form arc-shaped creases 61.

Further, the welded-portion row Ra in the first welded region A1 has the convex portions having the same shape (the same height and gradient), and the convex portions protruding toward the one side in the lateral direction and the convex portions protruding toward the other side in the lateral direction are arranged alternately in the vertical direction. Accordingly, the convex portions in the welded-portion row Ra are partially overlapped, in the lateral direction, with the welded-portion row Ra adjacent thereto in the lateral direction.

For example, as shown in FIG. 7, the second convex portion 52 is located on the one side in the lateral direction with respect to a virtual line Ld extending downward along the vertical direction from the lateral one-side end of the welded portion 503, which is located furthest on the other side in the lateral direction in the third convex portion 53. Thus, below the arc-shaped crease 61 formed between the fifth convex portion 55 and the third convex portion, the crease 61 having a shape of an arc protruding in a opposite direction (crease 61 extending along the second convex portion 52) is more easily formed.

As such, it is preferable that the welded-portion row Ra has a shape meandering in the lateral direction, and that the arc-shaped creases 61 that protrude toward the one side and the other side in the lateral direction are alternately formed in the vertical direction, as shown in FIGS. 9 and 10. According to this configuration, with the arc-shaped creases 61 changing in their directions, the creases 61 formed in the front waist portion 20 are divided in the vertical direction. This facilitates bending of the front waist portion 20 in the vertical direction, thereby increasing the flexibility of the front waist portion 20.

However, the shape of the welded portion row Ra is not limited to the above configuration. For example, only a part of the welded-portion row Ra may have the convex portion, and another part may have a linear shape, or may have convex portions with different shapes (heights and gradients).

Further, in the first welded region A1 of the present embodiment, the welded-portion rows Ra having the same arrangement pattern of the welded portions 50 are arranged side-by-side in the lateral direction at equal intervals D3. Thus, in the first welded region A1, the first welded-portion row Ra1 having the first convex portion 51 and the second convex portion 52 and the second welded-portion row Ra2 having the third convex portion 53 are alternately arranged in the lateral direction. In other words, the first welded region A1 has a plurality of combinations of the first welded-portion row Ra1 and the second welded-portion row Ra2 that are adjacent in the lateral direction.

Thus, in the first welded region A1, the relationship among the first convex portion 51 to the third convex portion 53 described above is established for multiple groups of the welded-portion rows Ra adjacent to each other in the lateral direction. Thus, in the first welded region A1, multiple arc-shaped creases 61 are more easily formed in the lateral direction, and thus the user is likely to have an impression that the front waist portion 20 is flexible. Note that the above-described third welded-portion row Ra3 corresponds to the first welded-portion row Ra1 as well, and the fourth convex portion 54 in the third welded-portion row Ra3 corresponds to the fifth convex portion 55 in the first welded-portion row Ra1.

More preferably, the first welded region A1 is overlapped, in the lateral direction, with at least a part of the side joining portion SS on the one side in the lateral direction, and is overlapped, in the lateral direction, with at least a part of the side joining portion SS on the other side in the lateral direction, as shown in FIG. 4. In FIG. 4, the welded-portion rows Ra are formed up to parts on the respective outer sides in the lateral direction with respect to the joining portions SS.

In this case, the arc-shaped creases 61 are more easily formed over a wide range in the lateral direction in the front waist portion 20. Thus, the arc-shaped creases 61 are likely to be recognized by the user, and the user is likely to have an impression that the front waist portion 20 is flexible.

Further, it is preferable that the welded-portion rows Ra having the same arrangement pattern of the welded portions 50 are arranged side-by-side at equal intervals D3 in the lateral direction, as in the first welded region A1 in the present embodiment. According to this configuration, more arc-shaped creases 61 are easily formed. Note that the same arrangement pattern and the equal intervals D3 refer to equality in terms of design, and include slight deviations caused by manufacturing errors. However, there is no limitation to the above configuration, it is sufficient that the first welded region A1 has at least one, and preferably multiple, combinations of the first welded-portion row Ra1 and the second welded-portion row Ra2, and the first welded region A1 may have welded-portion rows of different shapes or at different intervals between the welded-portion rows. In this case, the range from the first welded-portion row Ra1 or the second welded-portion row Ra2 located furthest on the one side in the lateral direction to the first welded-portion row Ra1 or the second welded-portion row Ra2 located furthest on the other side is defined as the first welded region A1.

Further, when the skin-side sheet 21 and the non-skin side sheet 22 (a pair of sheets) constituting the front waist portion 20 (the stretchy sheet 40) are made of nonwoven fabric, this nonwoven fabric may be subjected to embossing. In this case, the shape of one embossing pattern is preferably a shape with its lateral length that is less than or equal to its vertical length (e.g., a dot or square, or a diamond, oval, or rectangle that is longer in the vertical direction). According to this configuration, when the front waist portion 20 contracts in the lateral direction, the sheet between the welded-portion rows Ra bulges outward in the thickness direction, which makes it easier to form the arc-shaped crease 61.

### Second welded region A2

Next, the second welded region A2 will be described. As shown in FIG. 4, the second welded region A2 is located on the upper side with respect to the first welded region A1 and is located in the upper end portion of the front waist portion 20 with a predetermined width. The upper end portion of the waist portion refers to the upper region when the waist portion is divided into two in the vertical direction, and preferably refers to the uppermost region when the waist portion is divided into three in the vertical direction. FIG. 4 shows an example of the case where the second welded region A2 is located in a region having a predetermined width W1 (e.g., about 30 mm) from the upper end 20a of the front waist portion 20, but there is no limitation to this configuration. For example, the second welded region A2 may be located at a position spaced apart downward from the upper end 20a of the front waist portion 20.

The welded-portion rows Rb (fourth welded-portion row) in the second welded region A2, similarly to the welded-portion rows Ra of the first welded region A1, each have convex portions that protrude alternately toward the one side and the other side in the lateral direction, and form a shape meandering in the lateral direction. However, it is preferable that they are arranged in the lateral direction at the intervals D4 (< D3) smaller than the lateral intervals D3 of the welded-portion rows Ra in the first welded region A1.

Furthermore, it is preferable that when the front waist portion 20 is stretched in the lateral direction, any of the following is applied. First, as shown in FIG. 8A, it is preferable that the length of an overlap between the welded-portion rows Rb adjacent to each other in the lateral direction is less than or equal to a lateral length h of the welded portion 50 (e.g., the welded portion 506 and the welded portion 507) located on the most protruding side of the convex portion in the welded-portion rows Rb. In other words, it is preferable that in the convex portions 56, 57 that protrude toward the one side in the lateral direction and that are adjacent to each other in the vertical direction in the welded-portion row Rb on the other side in the lateral direction, the line connecting the lateral other-side ends of the welded portions 506 and 507 that are located furthest on the one side in the lateral direction is defined as a virtual line Le. In this case, a part of the convex portion 58 that protrudes toward the other side in the lateral direction and that is included in the welded-portion row Rb located adjacent to the welded-portion row Rb on the one side in the lateral direction is overlapped with the convex portions 56, 57, but the part is preferably located on the outer side with respect to the virtual line Le (on the one side in the lateral direction).

Alternatively, as shown in FIG. 8B, it is preferable that the welded-portion rows Rb adjacent to each other in the lateral direction are not overlapped in the lateral direction. In other words, it is preferable that the lateral one-side end (convex portion 56, 57) of the welded-portion row Rb located on the other side in the lateral direction is aligned in the lateral direction with or separated in the lateral direction from the lateral other-side end of the welded-portion row Rb (convex portion 58) located on the one side in the lateral direction.

In the above case, the amount of meandering of the convex portions 56 to 58 in the welded-portion row Rb tends to be small, and the gradients of the convex portions 56 to 58 tend to be small. Thus, as shown in FIG. 9, when the front waist portion 20 contracts, the creases 62 formed between the welded-portion rows Rb result in creases (creases closer to vertical creases) with a smaller slope with respect to the vertical direction, as compared to the arc-shaped creases 61 formed in the first welded region A1. In the second welded region A2, such creases 62 are formed at intervals smaller than the intervals of the creases 61 formed in the first welded region A1.

Accordingly, the user who see the second welded region A2 are more likely to have an impression that it has a better fit than the first welded region A1. With provision of such second welded region A2 at the upper end portion of the front waist portion 20, the user is more likely to have an impression that the waist upper portion (the second welded region A2 tightly fits and is less likely to slip downward while other part (the first welded region A1) fits softly and is comfortable.

Further, in the second welded region A2, the creases 62 close to vertical creases are formed, thereby increasing the stiffness in the vertical direction in the second welded region A2. This also prevents curling in the upper end portion of the front waist portion 20 due to the roundness of the wearer's stomach region and the like. However, there is no limitation to the above configuration, and the front waist portion 20 (the stretchy sheet 40) does not have to have the second welded region A2.

### Other embodiments

In the embodiment described above, the stretchy sheet 40 is provided to the front waist portion 20. In other words, the stretchy sheet 40 is provided to the front waist portion 20 so that the lateral direction of the stretchy sheet 40 (the direction along which the elastic members extend) is aligned with the lateral direction of the underpants-shaped diaper 1, and the vertical direction of the stretchy sheet 40 (the direction perpendicular to the elastic members) is aligned with the vertical direction of the underpants-shaped diaper 1. Further, in the above embodiment, both the front waist portion 20 and the back waist portion 30 are configured with the stretchy sheet 40, but only one of the waist portions may be configured with the stretchy sheet 40. Further, as in the back waist portion 30, when an extension portion 30E is provided below the side joining portions SS, and elastic members are arranged along the lateral direction in the extension portion 30E as well, which is different from FIG. 2, the extension portion 30E may also be configured with the stretchy sheet 40.

Further, the stretchy sheet 40 is not limited to being applied to the waist portion 20, 30 of the underpants-shaped diaper 1, but may also be applied to other parts of the diaper 1, such as leg elastic portions and the like. Further, the stretchy sheet 40 may be applied to an absorbent article (e.g., a tape-type diaper, a urine absorbing pad, a sanitary product, etc.) other than the underpants-shaped diaper 1, or may be applied to a product (e.g., a cleaning material, a mask, etc.) other than the absorbent article.

Embodiments of the present invention have been described above, however, embodiments of the present disclosure described above are simply to facilitate understanding of the present disclosure and are not in any way to be construed as limiting the present disclosure_{∘} The present disclosure may variously be changed or altered without departing from its essential features and encompass equivalents thereof.

**[Reference Signs List]**

| | | | | | |
|---|---|---|---|---|---|
| 1 | diaper (underpants-shaped absorbent article), | | | | |
| 10 | absorbent main body, | 11 | absorbent core, | 12 | top sheet, |
| 13 | back sheet, | 14 | exteri or sheet, | 15 | leg elastic member, |
| 16 | leak-proof wall elastic member, | | | | |
| 20 | front waist portion (waist portion), | | | | |
| 21 | skin-side sheet (sheet), | 22 | non-skin side sheet (sheet), | | |
| 23 | waist elastic member (elastic member) | | | | |
| 30 | back waist portion (waist portion), | | | | |
| 30E | extension portion, | | | | |
| 31 | skin-side sheet (sheet), | 32 | non-skin side sheet (sheet), | | |
| 33 | waist elastic member (elastic member), | | | | |
| 40 | stretchy sheet, | | | | |
| SS | side joining portion, | | | | |
| 50 | welded portion, | 50S | welded portion pair, | | |
| 51 | first convex portion, | 52 | second convex portion, | | |
| 53 | third convex portion, | | | | |
| La | virtual line (line), | Lb | virtual line (another line), | | |
| P1 | first intersection, | P2 | second intersection, | | |
| 61 | crease, | 62 | crease | | |

## Claims

1. A stretchy sheet (40) having a vertical direction and a lateral direction intersecting each other, the stretchy sheet (40) comprising:
a pair of sheets (21, 22);
a plurality of welded portions (50) joining the pair of sheets (21, 22);
a plurality of elastic members (23) capable of stretching and contracting in the lateral direction,
the plurality of elastic members (23) being arranged with a space in the vertical direction and between the pair of sheets (21, 22) that are joined by the plurality of welded portions (50),
the elastic members (23), in a state of contracting in the lateral direction, being each sandwiched between two welded portions (50s) adjacent to each other in the vertical direction, thereby restricting positions of the elastic members (23) in the lateral direction with respect to the pair of sheets (21, 22), the two welded portions (50s) included in the plurality of welded portions (50); and
a plurality of welded-portion rows (Ra) each having the plurality of welded portions (50) that are arranged side-by-side in the vertical direction,
the plurality of welded-portion rows (Ra) including a first welded-portion row (Ra1) and a second welded-portion row (Ra2) that is located adjacent to the first welded-portion row (Ra1) on one side in the lateral direction,
the first welded-portion row (Ra1) having a first convex portion (51) and a second convex portion (52) that protrude toward the one side in the lateral direction and that are adjacent to each other in the vertical direction,
the second welded-portion row (Ra2) having a third convex portion (53) that protrudes toward another side in the lateral direction,
in a state where the stretchy sheet (40) is stretched in the lateral direction, at least a part of a welded portion (503) located furthest on the other side in the lateral direction in the third convex portion (53) is located on the other side in the lateral direction with respect to a line (La) connecting lateral other-side ends (501, 502) of welded portions located furthest on the one side in the lateral direction in the first convex portion (51) and the second convex portion (52), the welded portion (503) and the welded portions (501, 502) being included in the plurality of welded portions (50).

2. The stretchy sheet (40) according to claim 1, wherein
the plurality of welded-portion rows (Ra) include a third welded-portion row (Ra3) that is located adjacent to the second welded-portion row (Ra2) on the one side in the lateral direction,
the third welded-portion row (Ra3) has a fourth convex portion (54) that protrudes toward the other side in the lateral direction, and
in the state where the stretchy sheet (40) is stretched in the lateral direction, a welded portion (504) located furthest on the other side in the lateral direction in the fourth convex portion (54) is located on the one side in the lateral direction with respect to the line (La), the welded portion (504) being included in the plurality of welded portions (50).

3. The stretchy sheet (40) according to claim 1 or 2, wherein
in the state where the stretchy sheet (40) is stretched in the lateral direction,
a half or more of the welded portion (503) in the lateral direction located furthest on the other side in the lateral direction in the third convex portion (53) is located on the other side in the lateral direction with respect to the line (La).

4. The stretchy sheet (40) according to claim 3, wherein
in the state where the stretchy sheet (40) is stretched in the lateral direction,
entirety of the welded portion (503) located furthest on the other side in the lateral direction in the third convex portion (53) is located on the other side in the lateral direction with respect to the line (La).

5. The stretchy sheet (40) according to claim 1 or 2, wherein
in the state where the stretchy sheet (40) is stretched in the lateral direction,
a lateral interval (D3) between the first welded-portion row (Ra1) and the second welded-portion row (Ra2) is equal to or more than 6 mm.

6. The stretchy sheet (40) according to claim 1 or 2, wherein
the first convex portion (51) is located on an upper side in the vertical direction with respect to the second convex portion (52),
the stretchy sheet (40) has,
in the state of being stretched in the lateral direction,
another line (Lb) connecting lateral other-side ends of the plurality of welded portions (50) constituting the second welded-portion row (Ra2),
a first intersection (P1) at which the other line (Lb) and the line (La) intersect each other on the upper side, and
a second intersection (P2) at which the other line (Lb) and the line (La) intersect each other on a lower side in the vertical direction, and
a vertical length (W2) from the lateral other-side end of the welded portion (501) located furthest on the one side in the lateral direction in the first convex portion (51) to the first intersection (P1) is longer than a vertical length (W3) from the first intersection (P1) to the second intersection (P2).

7. The stretchy sheet (40) according to claim 1 or 2, wherein
the plurality of elastic members (23) include a first elastic member (231) and a second elastic member (232) that is located adjacent to the first elastic member on a lower side in the vertical direction, and
a welded portion (50l) on the lower side of the two welded portions sandwiching the first elastic member (231) in the vertical direction is non-continuous with a welded portion (50u) on an upper side of the two welded portions sandwiching the second elastic member (232) in the vertical direction.

8. An underpants-shaped absorbent article (1) having the stretchy sheet (40) according to claim 1 or 2,
the underpants-shaped absorbent article (1) having a vertical direction and a lateral direction intersecting each other, the underpants-shaped absorbent article (1) comprising
an absorbent main body (10); and
a pair of waist portions (20, 30),
at least one of the pair of waist portions (20, 30) including the stretchy sheet (40),
the vertical direction of the stretchy sheet (40) being aligned with the vertical direction of the underpants-shaped absorbent article (1),
the lateral direction of the stretchy sheet (40) being aligned with the lateral direction of the underpants-shaped absorbent article (1).

9. The underpants-shaped absorbent article (1) according to claim 8, wherein
the stretchy sheet (40) includes a first welded region (A1), the first welded region (A1) including a plurality of combinations of the first welded-portion row (Ra1) and the second welded-portion row (Ra2) that are adjacent to each other in the lateral direction.

10. The underpants-shaped absorbent article (1) according to claim 9, wherein
the pair of waist portions (20, 30) are joined, at lateral side portions thereof, by a pair of side joining portions (SS), and
the first welded region (A1) is
overlapped, in the lateral direction, with at least a part of one of the pair of the side joining portions (SS) on the one side in the lateral direction, and
overlapped, in the lateral direction, with at least a part of another of the pair of the side joining portions (SS) on the other side in the lateral direction.

11. The underpants-shaped absorbent article (1) according to claim 9 or 10, wherein
the stretchy sheet (40) further includes a second welded region (A2),
in the second welded region (A2), a plurality of fourth welded-portion rows (Rb) are arranged in the lateral direction, the plurality of fourth welded-portion rows (Rb) each having convex portions (56, 57, 58) that protrude alternately toward the one side and the other side in the lateral direction,
in a state where the underpants-shaped absorbent article (1) is stretched in the lateral direction,
the fourth welded-portion rows (Rb) adjacent to each other in the lateral direction are not overlapped with each other in the lateral direction or a length of an overlap therebetween in the lateral direction is less than or equal to a lateral length of a welded portion (506) located furthest on a protruding side in the lateral direction in the convex portions, the welded portion (506) being included in the plurality of welded portions (50),
a lateral interval (D4) between the fourth welded-portion rows (Rb) is smaller than a lateral interval (D3) between the first welded-portion row (Ra1) and the second welded-portion row (Ra2),
the second welded region (A2) is located in an upper end portion of the waist portion (20, 30) with a predetermined width, and
the first welded region (A1) is located on a lower side with respect to the second welded region (A2).

## Patentansprüche

1. Dehnbare Lage (40), die eine vertikale Richtung und eine laterale Richtung aufweist, die einander schneiden, wobei die dehnbare Lage (40) Folgendes umfasst:
ein Paar von Lagen (21, 22);
eine Vielzahl von verschweißten Teilen (50), die das Paar von Lagen (21, 22) verbinden;
eine Vielzahl von elastischen Elementen (23), die sich in der lateralen Richtung dehnen und zusammenziehen können,
wobei die Vielzahl von elastischen Elementen (23) mit einem Abstand in der vertikalen Richtung und zwischen dem Paar von Lagen (21, 22), die durch die Vielzahl von verschweißten Teilen (50) verbunden sind, angeordnet sind,
die elastischen Elemente (23) in einem zusammengezogenen Zustand in der lateralen Richtung jeweils zwischen zwei verschweißten Teilen (50s) benachbart zueinander in der vertikalen Richtung eingefügt sind, wodurch Positionen der elastischen Elemente (23) in der lateralen Richtung in Bezug auf das Paar von Lagen (21, 22) eingeschränkt sind, wobei die zwei verschweißten Teile (50s) in der Vielzahl von verschweißten Teilen (50) eingeschlossen sind; und
eine Vielzahl von Reihen von verschweißten Teilen (Ra), die jeweils eine Vielzahl von verschweißten Teilen (50) aufweisen, die in der vertikalen Richtung nebeneinander angeordnet sind,
wobei die Vielzahl von Reihen von verschweißten Teilen (Ra) Folgendes einschließt: eine erste Reihe von verschweißten Teilen (Ral) und eine zweite Reihe von verschweißten Teilen (Ra2), die sich benachbart zu der ersten Reihe von verschweißten Teilen (Ral) auf einer Seite in der lateralen Richtung befindet,
die erste Reihe von verschweißten Teilen (Ral) einen ersten konvexen Teil (51) und einen zweiten konvexen Teil (52) aufweist, die zu der einen Seite in der lateralen Richtung vorstehen und die in der vertikalen Richtung benachbart zueinander vorliegen,
die zweite Reihe von verschweißten Teilen (Ra2) einen dritten konvexen Teil (53) aufweist, der zu der anderen Seite in der lateralen Richtung vorsteht,
wobei in einem Zustand, in dem die dehnbare Lage (40) in der lateralen Richtung gedehnt ist, sich zumindest ein Teil eines verschweißten Teils (503), der sich am weitesten auf der anderen Seite in der lateralen Richtung in dem dritten konvexen Teil (53) befindet, auf der anderen Seite in der lateralen Richtung in Bezug auf eine Linie (La) befindet, die laterale Enden der anderen Seite (501, 502) von verschweißten Teilen, die sich am weitesten auf der einen Seite in der lateralen Richtung in dem ersten konvexen Teil (51) und dem zweiten konvexen Teil (52) befinden, verbinden, wobei der verschweißte Teil (503) und die verschweißten Teile (501, 502) in der Vielzahl von verschweißten Teilen (50) eingeschlossen sind.

2. Dehnbare Lage (40) nach Anspruch 1, wobei
die Vielzahl von Reihen von verschweißten Teilen (Ra) eine dritte Reihe von verschweißten Teilen (Ra3) einschließt, die sich benachbart zu der zweiten Reihe von verschweißten Teilen (Ra2) auf der einen Seite in der lateralen Richtung befindet,
die dritte Reihe von verschweißten Teilen (Ra3) einen vierten konvexen Teil (54) aufweist, der zu der anderen Seite in der lateralen Richtung vorsteht, und
in dem Zustand, in dem die dehnbare Lage (40) in der lateralen Richtung gedehnt ist, sich ein verschweißter Teil (504), der sich am weitesten auf der anderen Seite in der lateralen Richtung in dem vierten konvexen Teil (54) befindet, auf der einen Seite in der lateralen Richtung in Bezug auf die Linie (La) befindet, wobei der verschweißte Teil (504) in der Vielzahl von verschweißten Teilen (50) eingeschlossen ist.

3. Dehnbare Lage (40) nach Anspruch 1 oder 2, wobei
in dem Zustand, in dem die dehnbare Lage (40) in der lateralen Richtung gedehnt ist,
eine Hälfte oder mehr des verschweißten Teils (503) in der lateralen Richtung, der sich am weitesten auf der anderen Seite in der lateralen Richtung in dem dritten konvexen Teil (53) befindet, auf der anderen Seite in der lateralen Richtung in Bezug auf die Linie (La) befindet.

4. Dehnbare Lage (40) nach Anspruch 3, wobei
in dem Zustand, in dem die dehnbare Lage (40) in der lateralen Richtung gedehnt ist,
sich die Gesamtheit des verschweißten Teils (503), der sich am weitesten auf der anderen Seite in der lateralen Richtung in dem dritten konvexen Teil (53) befindet, auf der anderen Seite in der lateralen Richtung in Bezug auf die Linie (La) befindet.

5. Dehnbare Lage (40) nach Anspruch 1 oder 2, wobei
in dem Zustand, in dem die dehnbare Lage (40) in der lateralen Richtung gedehnt ist,
ein lateraler Abstand (D3) zwischen der ersten Reihe von verschweißten Teilen (Ral) und der zweiten Reihe von verschweißten Teilen (Ra2) gleich oder größer als 6 mm ist.

6. Dehnbare Lage (40) nach Anspruch 1 oder 2, wobei
sich der erste konvexe Teil (51) auf einer oberen Seite in der vertikalen Richtung in Bezug auf den zweiten konvexen Teil (52) befindet,
die dehnbare Lage (40) Folgendes aufweist:
in dem Zustand der Dehnung in der lateralen Richtung
eine weitere Linie (Lb), die laterale Enden der anderen Seite der Vielzahl von verschweißten Teilen (50), die die zweite Reihe von verschweißten Teilen (Ra2) darstellen, verbinden,
einen ersten Schnittpunkt (P1), an dem sich die andere Linie (Lb) und die Linie (La) auf der oberen Seite einander schneiden, und
einen zweiten Schnittpunkt (P2), an dem sich die andere Linie (Lb) und die Linie (La) auf einer unteren Seite in der vertikalen Richtung einander schneiden,
und
eine vertikale Länge (W2) von dem lateralen Ende der anderen Seite des verschweißten Teils (501), das sich am weitesten auf der einen Seite in der lateralen Richtung in dem ersten konvexen Teil (51) befindet, zu dem ersten Schnittpunkt (P1) länger ist als eine vertikale Länge (W3) von dem ersten Schnittpunkt (P1) zu dem zweiten Schnittpunkt (P2).

7. Dehnbare Lage (40) nach Anspruch 1 oder 2, wobei
die Vielzahl von elastischen Elementen (23) Folgendes einschließt: ein erstes elastisches Element (231) und ein zweites elastisches Element (232), das sich benachbart zu dem ersten elastischen Element auf einer unteren Seite in der vertikalen Richtung befindet, und
ein verschweißter Teil (501) auf der unteren Seite der zwei verschweißten Teile, die das erste elastische Element (231) in der vertikalen Richtung eingefügt halten, mit einem verschweißten Teil (50u) auf einer oberen Seite der zwei verschweißten Teile, die das zweite elastische Element (232) in der vertikalen Richtung eingefügt halten, nicht kontinuierlich vorliegt.

8. Unterhosen-förmiger absorbierender Artikel (1), der die dehnbare Lage (40) nach Anspruch 1 oder 2 aufweist,
wobei der Unterhosen-förmige absorbierende Artikel (1) eine vertikale Richtung und eine laterale Richtung aufweist, die einander schneiden, der Unterhosen-förmige absorbierende Artikel (1) Folgendes umfasst:
einen absorbierenden Hauptkörper (10); und
ein Paar von Taillenteilen (20, 30),
wobei mindestens einer des Paars von Taillenteilen (20, 30) die dehnbare Lage (40) einschließt,
die vertikale Richtung der dehnbaren Lage (40) mit der vertikalen Richtung des Unterhosen-förmigen absorbierenden Artikels (1) ausgerichtet ist,
die laterale Richtung der dehnbaren Lage (40) mit der lateralen Richtung des Unterhosen-förmigen absorbierenden Artikels (1) ausgerichtet ist.

9. Unterhosen-förmiger absorbierender Artikel (1) nach Anspruch 8, wobei
die dehnbare Lage (40) einen ersten verschweißten Bereich (A1) einschließt, wobei der erste verschweißte Bereich (A1) eine Vielzahl von Kombinationen der ersten Reihe von verschweißten Teilen (Ral) und der zweiten Reihe von verschweißten Teilen (Ra2) einschließt, die benachbart zueinander in der lateralen Richtung vorliegen.

10. Unterhosen-förmiger absorbierender Artikel (1) nach Anspruch 9, wobei
das Paar von Taillenteilen (20, 30) an lateralen Seitenteilen davon durch ein Paar von Seitenverbindungsteilen (SS) verbunden sind und
der erste verschweißte Bereich (A1) Folgendes ist:
in der lateralen Richtung mit mindestens einem Teil von einem des Paars der Seitenverbindungsteile (SS) auf der einen Seite in der lateralen Richtung überlappt und
in der lateralen Richtung mit mindestens einem Teil von einem anderen des Paars der Seitenverbindungsteile (SS) auf der anderen Seite in der lateralen Richtung überlappt.

11. Unterhosen-förmiger absorbierender Artikel (1) nach Anspruch 9 oder 10, wobei
die dehnbare Lage (40) weiter einen zweiten verschweißten Bereich (A2) einschließt,
in dem zweiten verschweißten Bereich (A2) eine Vielzahl von vierten Reihen von verschweißten Teilen (Rb) in der lateralen Richtung angeordnet ist, wobei die Vielzahl von vierten Reihen von verschweißten Teilen (Rb) jeweils konvexe Teile (56, 57, 58) aufweist, die abwechselnd zu der einen Seite und der anderen Seite in der lateralen Richtung vorstehen,
in einem Zustand, in dem der Unterhosen-förmige absorbierende Artikel (1) in der lateralen Richtung gedehnt ist,
die vierten Reihen von verschweißten Teilen (Rb) in der lateralen Richtung benachbart zueinander nicht miteinander in der lateralen Richtung überlappen oder eine Länge einer Überlappung dazwischen in der lateralen Richtung kleiner ist als oder gleich einer lateralen Länge eines verschweißten Teils (506), das sich am weitesten auf einer vorstehenden Seite in der lateralen Richtung in den konvexen Teilen befindet, wobei der verschweißte Teil (506) in der Vielzahl von verschweißten Teilen (50) eingeschlossen ist,
ein lateraler Abstand (D4) zwischen den vierten Reihen von verschweißten Teilen (Rb) kleiner ist als ein lateraler Abstand (D3) zwischen der ersten Reihe von verschweißten Teilen (Ral) und der zweiten Reihe von verschweißten Teilen (Ra2),
sich der zweite verschweißte Bereich (A2) in einem oberen Endteil des Taillenteils (20, 30) mit einer vorgegebenen Breite befindet und
sich der erste verschweißte Bereich (A1) auf einer unteren Seite in Bezug auf den zweiten verschweißten Bereich (A2) befindet.

## Revendications

1. Feuille extensible (40) ayant une direction verticale et une direction latérale qui se croisent l'une par rapport à l'autre, la feuille extensible (40) comportant :
une paire de feuilles (21, 22) ;
une pluralité de parties soudées (50) permettant d'assembler la paire de feuilles (21, 22) ;
une pluralité d'éléments élastiques (23) en mesure de s'étirer et de se contracter dans la direction latérale,
les éléments de la pluralité d'éléments élastiques (23) étant agencés avec un espace dans la direction verticale et entre la paire de feuilles (21, 22) qui sont assemblées par la pluralité de parties soudées (50),
les éléments élastiques (23), dans un état de contraction dans la direction latérale, étant chacun pris en sandwich entre deux parties soudées (50s) adjacentes l'une par rapport à l'autre dans la direction verticale, pour de ce fait limiter les positions des éléments élastiques (23) dans la direction latérale par rapport à la paire de feuilles (21, 22), les deux parties soudées (50s) étant incluses dans la pluralité de parties soudées (50) ; et
une pluralité de rangées de parties soudées (Ra) ayant chacune la pluralité de parties soudées (50) qui sont agencées côte à côte dans la direction verticale,
la pluralité de rangées de parties soudées (Ra) comprenant une première rangée de parties soudées (Ral) et une deuxième rangée de parties soudées (Ra2) qui est située de manière adjacente par rapport à la première rangée de parties soudées (Ra1) sur un côté dans la direction latérale,
la première rangée de parties soudées (Ra1) ayant une première partie convexe (51) et une deuxième partie convexe (52) qui font saillie vers ledit un côté dans la direction latérale et qui sont adjacentes l'une par rapport à l'autre dans la direction verticale,
la deuxième rangée de parties soudées (Ra2) ayant une troisième partie convexe (53) qui fait saillie vers un autre côté dans la direction latérale,
dans un état dans lequel la feuille extensible (40) est étirée dans la direction latérale, au moins une partie d'une partie soudée (503) située le plus loin sur ledit autre côté dans la direction latérale dans la troisième partie convexe (53) est située sur ledit autre côté dans la direction latérale par rapport à une ligne (La) reliant les extrémités dudit autre côté (501, 502) de parties soudées situées le plus loin sur ledit un côté dans la direction latérale dans la première partie convexe (51) et dans la deuxième partie convexe (52), la partie soudée (503) et les parties soudées (501, 502) étant incluses dans la pluralité de parties soudées (50).

2. Feuille extensible (40) selon la revendication 1, dans laquelle
les rangées de la pluralité de rangées de parties soudées (Ra) comprennent une troisième rangée de parties soudées (Ra3) qui est située de manière adjacente par rapport à la deuxième rangée de parties soudées (Ra2) sur ledit un côté dans la direction latérale,
la troisième rangée de parties soudées (Ra3) a une quatrième partie convexe (54) qui fait saillie vers ledit autre côté dans la direction latérale, et
dans l'état dans lequel la feuille extensible (40) est étirée dans la direction latérale, une partie soudée (504) située le plus loin sur ledit autre côté dans la direction latérale dans la quatrième partie convexe (54) est située sur ledit un côté dans la direction latérale par rapport à la ligne (La), la partie soudée (504) étant incluse dans la pluralité de parties soudées (50).

3. Feuille extensible (40) selon la revendication 1 ou la revendication 2, dans laquelle
dans l'état dans lequel la feuille extensible (40) est étirée dans la direction latérale,
une moitié ou plus de la partie soudée (503) dans la direction latérale située le plus loin sur ledit autre côté dans la direction latérale dans la troisième partie convexe (53) est située sur ledit autre côté dans la direction latérale par rapport à la ligne (La).

4. Feuille extensible (40) selon la revendication 3, dans laquelle
dans l'état dans lequel la feuille extensible (40) est étirée dans la direction latérale,
la totalité de la partie soudée (503) située le plus loin sur ledit autre côté dans la direction latérale dans la troisième partie convexe (53) est située sur ledit autre côté dans la direction latérale par rapport à la ligne (La).

5. Feuille extensible (40) selon la revendication 1 ou la revendication 2, dans laquelle
dans l'état dans lequel la feuille extensible (40) est étirée dans la direction latérale,
un intervalle latéral (D3) entre la première rangée de parties soudées (Ral) et la deuxième rangée de parties soudées (Ra2) est égal ou supérieur à 6 mm.

6. Feuille extensible (40) selon la revendication 1 ou la revendication 2, dans laquelle
la première partie convexe (51) est située sur un côté supérieur dans la direction verticale par rapport à la deuxième partie convexe (52),
la feuille extensible (40) a,
dans l'état dans lequel elle est étirée dans la direction latérale,
une autre ligne (Lb) reliant les extrémités latérales dudit autre côté de la pluralité de parties soudées (50) constituant la deuxième rangée de parties soudées (Ra2),
une première intersection (P1) au niveau de laquelle l'autre ligne (Lb) et la ligne (La) se croisent l'une par rapport à l'autre sur le côté supérieur, et
une deuxième intersection (P2) au niveau de laquelle l'autre ligne (Lb) et la ligne (La) se croisent l'une par rapport à l'autre sur un côté inférieur dans la direction verticale, et
une longueur verticale (W2) allant de l'extrémité latérale dudit autre côté de la partie soudée (501) située le plus loin sur ledit un côté dans la direction latérale dans la première partie convexe (51) à la première intersection (P1) est plus longue qu'une longueur verticale (W3) allant de la première intersection (P1) à la deuxième intersection (P2).

7. Feuille extensible (40) selon la revendication 1 ou la revendication 2, dans laquelle
les éléments de la pluralité d'éléments élastiques (23) comprennent un premier élément élastique (231) et un deuxième élément élastique (232) qui est situé de manière adjacente par rapport au premier élément élastique sur un côté inférieur dans la direction verticale, et
une partie soudée (501) sur le côté inférieur des deux parties soudées prenant en sandwich le premier élément élastique (231) dans la direction verticale est non continue avec une partie soudée (50u) sur un côté supérieur des deux parties soudées prenant en sandwich le deuxième élément élastique (232) dans la direction verticale.

8. Article absorbant en forme de culotte (1) ayant la feuille extensible (40) selon la revendication 1 ou la revendication 2,
l'article absorbant en forme de culotte (1) ayant une direction verticale et une direction latérale se croisant l'une par rapport à l'autre, l'article absorbant en forme de culotte (1) comportant
un corps principal absorbant (10) ; et
une paire de parties au niveau de la taille (20, 30),
au moins l'une de la paire de parties au niveau de la taille (20, 30) comprenant la feuille extensible (40),
la direction verticale de la feuille extensible (40) étant alignée sur la direction verticale de l'article absorbant en forme de culotte (1),
la direction latérale de la feuille extensible (40) étant alignée sur la direction latérale de l'article absorbant en forme de culotte (1).

9. Article absorbant en forme de culotte (1) selon la revendication 8, dans lequel
la feuille extensible (40) comprend une première région soudée (Al), la première région soudée (Al) comprenant une pluralité de combinaisons de la première rangée de parties soudées (Ral) et de la deuxième rangée de parties soudées (Ra2) qui sont adjacentes l'une par rapport à l'autre dans la direction latérale.

10. Article absorbant en forme de culotte (1) selon la revendication 9, dans lequel
les parties de la paire de parties au niveau de la taille (20, 30) sont assemblées, au niveau des parties côté latéral de celles-ci, par une paire de parties latérales d'assemblage (SS), et
la première région soudée (Al) est
chevauchée, dans la direction latérale, par au moins une partie de l'une de la paire de parties latérales d'assemblage (SS) sur ledit un côté dans la direction latérale, et
chevauchée, dans la direction latérale, par au moins une partie d'une autre de la paire de parties latérales d'assemblage (SS) sur ledit autre côté dans la direction latérale.

11. Article absorbant en forme de culotte (1) selon la revendication 9 ou la revendication 10, dans lequel
la feuille extensible (40) comprend par ailleurs une deuxième région soudée (A2),
dans la deuxième région soudée (A2), les rangées d'une pluralité de quatrièmes rangées de parties soudées (Rb) sont disposées dans la direction latérale, les rangées de la pluralité de quatrièmes rangées de parties soudées (Rb) ayant chacune des parties convexes (56, 57, 58) qui font tour à tour saillie vers ledit un côté et ledit autre côté dans la direction latérale,
dans un état dans lequel l'article absorbant en forme de culotte (1) est étiré dans la direction latérale,
les quatrièmes rangées de parties soudées (Rb) adjacentes les unes par rapport aux autres dans la direction latérale ne se chevauchent pas les unes par rapport aux autres dans la direction latérale ou une longueur de chevauchement entre elles dans la direction latérale est inférieure ou égale à une longueur latérale d'une partie soudée (506) située le plus loin sur un côté saillant dans la direction latérale dans les parties convexes, la partie soudée (506) étant comprise dans la pluralité de parties soudées (50),
un intervalle latéral (D4) entre les quatrièmes rangées de parties soudées (Rb) est inférieur à un intervalle latéral (D3) entre la première rangée de parties soudées (Ral) et la deuxième rangée de parties soudées (Ra2),
la deuxième région soudée (A2) est située dans une partie d'extrémité supérieure de la partie au niveau de la taille (20, 30) avec une largeur prédéterminée, et
la première région soudée (Al) est située sur un côté inférieur par rapport à la deuxième région soudée (A2).
